(19) 

Europäisches
Patentamt
European
Patent Office
Office européen
des brevets

(11)  **EP 2 298 446 A1**

(12) **EUROPEAN PATENT APPLICATION**
published in accordance with Art. 153(4) EPC

(43) Date of publication:
**23.03.2011 Bulletin 2011/12**

(21) Application number: **09758232.4**

(22) Date of filing: **25.05.2009**

(51) Int Cl.:
*B01J 35/02* (2006.01)   *B01J 23/88* (2006.01)
*B01J 37/00* (2006.01)   *C07C 27/12* (2006.01)
*C07C 45/35* (2006.01)   *C07C 47/22* (2006.01)
*C07C 57/05* (2006.01)   *C07B 61/00* (2006.01)
*C07C 51/235* (2006.01)   *C07C 51/25* (2006.01)

(86) International application number:
**PCT/JP2009/059503**

(87) International publication number:
**WO 2009/147965 (10.12.2009 Gazette 2009/50)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(30) Priority: **02.06.2008 JP 2008144213
30.01.2009 JP 2009019750**

(71) Applicant: **Nippon Kayaku Kabushiki Kaisha
Tokyo 102-8172 (JP)**

(72) Inventors:
• **NAKAHARA Masaki
Sanyoonoda-shi
Yamaguchi 757-0002 (JP)**

• **SUGI Hideki
Sanyoonoda-shi
Yamaguchi 757-0002 (JP)**
• **SEO Yoshimasa
Takasaki-shi
Gunma 370-1208 (JP)**
• **KURAKAMI Tatsuhiko
Sanyoonoda-shi
Yamaguchi 757-0002 (JP)**
• **ONOUE Hiroyuki
Tokyo 102-8172 (JP)**

(74) Representative: **Gille Hrabal Struck Neidlein Prop
Roos
Patentanwälte
Brucknerstrasse 20
40593 Düsseldorf (DE)**

(54)  **CATALYST AND METHOD OF PRODUCING UNSATURATED ALDEHYDE AND UNSATURATED
CARBOXYLIC ACID**

(57)     Provided is a catalyst which can prevent a lowering in selectivity for a target product in a gas phase catalytic reaction and has an excellent frictional resistance. A catalyst which is a supported catalyst comprising an inert support that is coated with a catalyst powder, **characterized in that** the inert support is ring-shaped and has an outer periphery that is curved in the lengthwise direction of the support, and the catalyst is produced by granulation in a moisten environment. The above described catalyst is useful in the gas phase oxidation of propylene, isobutylene, tertiary-butyl alcohol or methyl tertiary-butyl ether to thereby produce an unsaturated aldehyde corresponding thereto, or in the gas phase oxidation of such an unsaturated aldehyde as described above to thereby produce an unsaturated carboxylic acid.

EP 2 298 446 A1

**Description**

Technical Field

The present invention relates to a novel catalyst used in a gas phase catalytic oxidation reaction for obtaining an unsaturated aldehyde or an unsaturated carboxylic acid.

Background Art

There have hitherto been a large number of suggestions on the active ingredient composition or shape in regard to catalysts for producing acrolein and acrylic acid by subjecting propylene to gas phase catalytic oxidation, catalysts for producing methacrolein and methacrylic acid by subjecting isobutylene, tertiary-butyl alcohol or methyl tertiary-butyl ether to gas phase catalytic oxidation, or catalysts for producing acrylic acid or methacrylic acid by subjecting acrolein or methacrolein to gas phase catalytic oxidation. For example, shapes of the catalyst such as a pellet form, a spherical form, a cylindrical form, and a ring form are known, and a catalyst is used after being molded in a form appropriate for the operating conditions for carrying out an oxidation reaction, and the production conditions and performance of the catalyst itself. Furthermore, in regard to the method for molding a catalyst, various methods such as tablet molding, extrusion molding and coating molding are known.

The above oxidation reaction is usually carried out by passing a raw material gas prepared by mixing the raw materials, oxygen and the like, through a reaction tube packed with a catalyst. However, in this case, there occur problems, such as that when it is attempted to flow more and more of the raw material gas, an increase in the pressure occurs, and the selectivity ratio for the target product decreases; and that local abnormal high temperature areas (hot spots) occur in the catalyst layer, resulting in a decrease in the catalyst life or a decrease in the selectivity ratio for the target product due to excessive oxidation reaction, and in a worst case, the occurrence of a runaway reaction.

Among the catalysts described above, since a catalyst having a ring form, that is, a hollow cylindrical form or a cylindrical form having a through-hole, has a through-hole, the shape is known to have a small pressure drop. However, the shape has a small mechanical strength, and thus in some cases, the pressure loss rather increases, because the catalyst undergoes powderization during catalyst packing or powderization during use.

In order to compensate such defects, for example, Patent Document 1 describes a method of fabricating the through-hole in the center or the external form of the catalyst into an elliptical shape.

This method is intended to suppress the reduction in the catalyst spacing or powderization to the minimum by providing areas of low mechanical strength in the catalyst and designing the catalyst to be disintegrated at these areas. However, the prevention of powderization occurring at the time of catalyst packing is insufficient even though such a technology is applied, and an enhancement of the frictional resistance of the catalyst is further demanded.

Patent Document 2 describes a molded catalyst in a hollow cylindrical form or a ring-like tablet form, which is characterized in that a catalyst end surface is curved in two directions toward the edge end of the outer side and toward the edge end of the center hole, has an effect of suppressing a pressure drop. The catalyst disclosed in Patent Document 2 is a catalyst used in the oxychlorination of ethylene to 1,2-dichloroethane, and the method for producing a catalyst, which is substantially disclosed in the same document, is a method of impregnating a support with a catalytically active substance, that is, a method of impregnating the pores of a support with a small amount of a catalytically active substance. Patent Document 2 does not have any descriptions on a catalyst produced by coating a support with a catalyst powder.

Furthermore, Patent Document 3 describes a method of incorporating an inorganic fiber into the constituent components of a catalyst. According to this method, the mechanical strength of the catalyst is enhanced, but since the catalyst component itself is molded, the distance of passage of the reactive gas is lengthened, and heat accumulation in the catalyst is increased along with the progress of the reaction. Heat accumulation in the catalyst may cause a decrease in the catalyst life or a runaway reaction, and therefore, there is a demand for a catalyst having a higher heat removal effect.

Patent Document 4 also discloses a catalyst in which the end surface of a hollow cylinder, to which a catalyst component is applied, is curved.

Furthermore, Patent Document 5 discloses a technique of drying in a temperature-regulated and moisture-regulated environment. However, Patent Document 5 is not related to a supported catalyst.

Prior Art Documents

Patent Documents
Patent Document 1: Japanese Patent Application Laid-Open (JP-A) No. 6-170232

Patent Document 2: JP-ANo. 2001-293376
Patent Document 3: JP-ANo. 2002-273229
Patent Document 4: JP-ANo. 61-141933
Patent Document 5: JP-ANo. 2008-207127

Disclosure of the Invention

Problem to be Solved by the Invention

An object of the present invention is to provide a catalyst which suppresses a decrease in the selectivity ratio for a target product in a gas phase catalytic reaction using a supported catalyst produced by coating a support with a catalyst powder, and has excellent frictional resistance.

Means for Solving Problem

The inventors of the present invention have found that although the frictional resistance of a catalyst is not improved only by simply coating a ring-shaped support with a catalyst powder, the problems described above can be solved by setting particular conditions for the shape of the support itself and the granulation environment, thus completing the present invention.

That is, the present invention relates to:

(1) a catalyst for producing an unsaturated aldehyde and an unsaturated carboxylic acid by correspondingly subjecting propylene, isobutylene, tertiary-butyl alcohol or methyl tertiary-butyl ether to gas phase catalytic oxidation, the catalyst being a supported catalyst having an inert support coated with a catalyst powder, wherein the inert support is ring-shaped and has an outer periphery that is curved in the lengthwise direction of the support;

(2) a catalyst for producing an unsaturated carboxylic acid by subjecting a corresponding unsaturated aldehyde to gas phase catalytic oxidation, the catalyst being a supported catalyst having an inert support coated with a catalyst powder, wherein the inert support is ring-shaped and has an outer periphery that is curved in the lengthwise direction of the support;

(3) a method of producing a catalyst for producing an unsaturated aldehyde and an unsaturated carboxylic acid by correspondingly subjecting propylene, isobutylene, tertiary-butyl alcohol or methyl tertiary-butyl ether to gas phase catalytic oxidation, the method including a step of coating an inert support with a catalyst powder to produce a supported catalyst, wherein the inert support is ring-shaped and has an outer periphery that is curved in the lengthwise direction of the support, and wherein the catalyst is produced in an atmosphere of the step of coating that has been regulated to have a weight absolute humidity of 0.01 or higher;

(4) a method of producing a catalyst for producing an unsaturated carboxylic acid by subjecting a corresponding unsaturated aldehyde to gas phase catalytic oxidation, the method including a step of coating an inert support with a catalyst powder to produce a supported catalyst, wherein the inert support is ring-shaped and has an outer periphery that is curved in the lengthwise direction of the support, and wherein the catalyst is produced in an atmosphere of the step of coating that has been regulated to have a weight absolute humidity of 0.01 1 or higher;

(5) a method of producing an unsaturated aldehyde and an unsaturated carboxylic acid, wherein propylene, isobutylene, tertiary-butyl alcohol or methyl tertiary-butyl ether is correspondingly subjected to gas phase catalytic oxidation, by using the catalyst according to item (1) or the catalyst obtainable by the method according to item (3); and

(6) a method of producing an unsaturated carboxylic acid, wherein an unsaturated aldehyde is correspondingly subjected to gas phase catalytic oxidation by using the catalyst according to item (2) or the catalyst obtainable by the method according to item (4).

Effect of the Invention

According to the present invention, there can be provided a catalyst which suppresses a decrease in the selectivity ratio of a target product in a gas phase catalytic reaction and has excellent frictional resistance. Furthermore, since the catalyst has a through-hole, the maximum reaction temperature (hot spot) can be made small.

Best Mode for Carrying out the Invention

The catalyst of the present invention can be obtained by supporting by coating a catalyst powder on an inert support having a particular shape. The catalyst powder can be obtained by a method known per se. For example, as described in JP-ANo.10-028877 or JP-ANo. 50-30815, there may be mentioned a method of drying a slurry obtained by dissolving or dispersing a compound containing a catalytically active element in water, by means of evaporation to dryness or spray drying and the like, and calcining the obtained dry powder as necessary, at or above 400°C.

The catalyst powder that can be used with preference in the present invention will be explained.

Examples of the catalyst powder for producing an unsaturated aldehyde and an unsaturated carboxylic acid by correspondingly subjecting propylene, isobutylene, tertiary-butyl alcohol or methyl tertiary-butyl ether to gas phase catalytic oxidation by means of molecular oxygen or a molecular oxygen-containing gas, include catalyst powders having a composition represented by the following formula (1) as described in JP-ANo. 10-028877:

$$Mo_aBi_bNi_cCo_dFe_fY_gZ_hO_x \qquad (1)$$

wherein Mo, Bi, Ni, Co and Fe represent molybdenum, bismuth, nickel, cobalt and iron, respectively; Y represents at least one element selected from tin, zinc, tungsten, chromium, manganese, magnesium, antimony and titanium; Z represents at least one element selected from potassium, rubidium, thallium and cesium; and a, b, c, d, f, g, h and x represent the numbers of atoms of molybdenum, bismuth, nickel, cobalt, iron, Y, Z and oxygen, respectively, such that a =12, b = 0.1 to 7, c + d = 0.5 to 20, f = 0.5 to 8, g = 0 to 2, h = 0 to 1, and x = value determined by the oxidation state of the various elements.

Furthermore, examples of the catalyst powder for producing acrylic acid by subjecting acrolein to gas phase catalytic oxidation, include catalyst powders having a composition represented by the following formula (2) as described in JP-ANo. 2001-79408:

$$Mo_{12}V_aW_bCu_cSb_dX_eY_fZ_gO_h \qquad (2)$$

wherein Mo, V, W, Cu, Sb and O represent molybdenum, vanadium, tungsten, copper, antimony and oxygen, respectively; X represents at least one element selected from the group consisting of alkali metals and thallium; Y represents at least one element selected from the group consisting of magnesium, calcium, strontium, barium and zinc; Z represents at least one element selected from the group consisting of niobium, cerium, tin, chromium, manganese, iron, cobalt, samarium, germanium, titanium and arsenic; a, b, c, d, e, f, g and h respectively represent the atomic ratios of the various elements, and with respect to 12 molybdenum atoms, a represents $0 < a \leq 10$, b represents $0 \leq b \leq 10$, c represents $0 < c \leq 6$, d represents $0 < d \leq 10$, e represents $0 \leq e \leq 0.5$, f represents $0 \leq f \leq 1$, g represents $0 \leq g \leq 6$, and h represents the number of oxygen atoms required to satisfy the atomic valences of the various components.

Examples of the catalyst powder for producing methacrylic acid by subjecting methacrolein to gas phase catalytic oxidation, include catalyst powders having a composition represented by the following formula (3) as described in JP-ANo. 55-79341:

$$M0_{12}V_aP_bCU_cAS_dX_eO_z \qquad (3)$$

or the following formula (4) as described in JP-ANo. 55-122734:

$$Mo_{12}V_aP_bCu_cAs_dX_eY_fO_h \qquad (4).$$

The material of the inert support used to obtain the catalyst of the present invention is not particularly limited, as long as the material has an activity of 20 or less when the activity of the catalyst powder for the applicable reaction is designated as 100. Examples of the material include α-alumina, silicon carbide, pumice, silica, zirconium oxide, and titanium oxide.

The support according to the present invention is ring-shaped, that is, has a hollow cylindrical shape or a cylindrical shape having a through-hole, and use is made of a support in which the outer periphery, that is, the two ends in the lengthwise direction of a hollow cylinder, is curved in the lengthwise direction, that is, the outer diameter of the cross-section in the center of the lengthwise direction is larger than the outer diameter of the cross-section at the two ends in the lengthwise direction. Such a support can be produced by planing the outer periphery of a commercially available ring-shaped support with a ball mill or a tumbling granulator. Furthermore, a clay-like raw material or a dried product thereof may be molded with a ring-shape extrusion molding machine, subsequently the outer periphery of the obtained extrusion product may be rounded with a tumbling granulator or the like, and then calcined.

In regard to the size of the support, a support in which the outer diameter of the cross-section in the center of the lengthwise direction is usually 3 to 30 mm, and preferably 4 to 20 mm, and the length is usually about 0.5 to 2 times the outer diameter of the cross-section in the center, is used. Also, a support in which the diameter of the through-hole is usually 0.1 to 0.8 times the outer diameter of the cross-section in the center, is used.

The aperture radius of the support having the outer periphery rounded is the apparent inner radius, and cannot be said to be equivalent to the inner radius obtainable when the clay-like raw material is molded with a ring-shape extrusion molding machine.

The degree of curvature at the outer periphery can be adjusted by regulating the material of the support, or the mechanical abrasive force of the tumbling granulator or the like, and a support in which the outer circumferential radius of curvature which is defined below is usually about 0.01 to 0.5 times, and preferably 0.05 to 0.4 times, the outer diameter in the center, is used. In the following, the outer circumferential radius of curvature / the outer diameter in the center may also be referred to as the curvature of the outer periphery.

The outer circumferential radius of curvature refers to the radius of the rounded portion in the corner portion of a hollow cylinder, and this is the radius of an elliptical or circular roundness that can be obtained by planing the outer periphery of the raw material support with a tumbling granulator or a ball mill. Furthermore, the outer diameter in the center refers to the outer diameter of a hollow cylinder.

The void content, water absorption ratio and porosity of the support also affect the performance. The void content of the support is preferably 20% or more and 70% or less, and more preferably 25% or more and 55% or less. The water absorption ratio of the support is preferably 5% or more and 50% or less, and more preferably 10% or more and 40% or less. The porosity of the support is preferably 50% or more and 90% or less, and more preferably 60% or more and 80% or less.

The method of calculating the void content and the water absorption ratio is as follows. The method of measurement conforms to JIS R2205.

$$\text{Void content (\%)} = (W3 - W1)/(W3 - W2) \times 100$$

$$\text{Water absorption ratio (\%)} = (W3 - W1)/W1 \times 100$$

W1: Dry weight (120°C x 60 minutes)
W2: Weight in water
W3: Water-saturated weight

The method for supporting by coating a catalyst powder on an inert support is not particularly limited, but an example may be, as described in JP-ANo. 10-028877, a method of tumbling granulating a catalyst powder together with a binder such as an alcohol, or the like. After the catalyst powder is supported as such, this supported catalyst is dried and if necessary, calcined at 440 to 650°C, and thereby the catalyst of the present invention is obtained. For the catalyst of the present invention, the supporting ratio of the catalyst powder is usually 20 to 80% by weight, and preferably 30 to 70% by weight (catalyst powder/catalyst).

In order to support a catalyst powder on a support, it is preferable to use a molding aid and/or a strength enhancing aid. Specific examples of the molding aid that can be used include crystalline cellulose, starch, and stearic acid. The amount of use of the molding aid is usually 30% by weight or less based on the catalyst powder. Specific examples of the strength enhancing aid that can be used include "cera-mic" fibers, carbon fibers, and whiskers. The amount of use of the strength enhancing aid is usually 30% by weight based on the catalyst powder. The molding aid and/or strength enhancing aid may be mixed in advance before supporting the catalyst powder, or may be added simultaneously with or before and after the addition of the catalyst powder and the like into a molding machine as will be described later.

Furthermore, in order to support the catalyst powder on a support, it is preferable to use a binder. Specific examples of the binder that can be used include water; a polymer binder such as polyvinyl alcohol; an inorganic binder such as silica sol or alumina sol; and an organic binder such as a polyhydric alcohol such as ethylene glycol or glycerin, or a mixture thereof

The amount of use of the binder is usually 10 to 60% by weight based on the catalyst powder.

The weight absolute humidity (kg-water vapor/kg-dry air) of the atmosphere upon coating is 0.01 or higher, preferably 0.01 to 0.05, and more preferably 0.015 to 0.04. In that case, the temperature is preferably 20°C or higher, and the relative humidity is preferably 20% or higher. Regulation of the atmosphere for coating may be achieved by a method of bringing the humidity in the granulation chamber to a certain value using an appropriate apparatus. Furthermore, if the atmosphere is humidified under the conditions described above when the molded catalyst is dried and/or calcined, the degree of wear remains equal or is slightly decreased, which is preferable.

The aperture diameter of the support and the catalyst is the apparent inner diameter, and cannot be said to be a uniform aperture diameter. The diameter may vary at the inlet and at the outlet, or the opening of one side or both sides may be closed.

The method of calculating the aperture diameter is as follows.

$$\text{Aperture diameter (mm)} = (\text{Aperture diameter at the inlet (mm)} + \text{aperture diameter at the outlet (mm)})/2$$

The catalyst of the present invention can be applied to a method of producing an unsaturated aldehyde and an unsaturated carboxylic acid by correspondingly subjecting propylene, isobutylene, tertiary-butyl alcohol or methyl tertiary-butyl ether to gas phase catalytic oxidation, or to a method of producing an unsaturated carboxylic acid by subjecting a corresponding unsaturated aldehyde to gas phase catalytic oxidation. The reaction can be carried out as a fixed-bed type gas phase catalytic oxidation reaction, and preferably a multitubular type gas phase catalytic oxidation reaction, and may be carried out by a conventional single-stream passage method or a recycling method. The reaction can be carried out under the conditions generally used. For example, considering an oxidation reaction of propylene, the reaction is carried out by introducing, as a raw material gas, a mixed gas formed from 1 to 10% by volume, and preferably 4 to 9% by volume, of propylene; 3 to 20% by volume, and preferably 4 to 18% by volume, of molecular oxygen; 0 to 60% by volume, and preferably 4 to 50% by volume, of water vapor; and 20 to 80% by volume, and preferably 30 to 60% by volume, of an inert gas (nitrogen, carbon dioxide, or the like), at 250 to 450°C and at a pressure of normal pressure to 10 atmospheric pressure, at a space velocity (= flow rate of raw material gas/apparent volume of packed catalyst) of 300 to 5000 hr$^{-1}$.

EXAMPLES

Hereinafter, the present invention will be described in more detail by way of Examples. The term parts used in the following means parts by weight, and the degree of wear, bulk specific gravity, porosity, conversion rate, selectivity ratio and yield are based on the following measurement methods or definitions.

(Degree of wear)

50 g of a catalyst is accurately weighed (this weight is designated as W6) and introduced into a plastic drum, having a diameter of 40 cm, of a tablet abrasion tester manufactured by Hayashi Rikagaku Co., Ltd. The catalyst is rotated at 25 rpm for 10 minutes. After completion of the test, the catalyst is sieved with a sieve having a mesh size of 2.38 mm, and the sample on the sieve is accurately weighed (this weight is designated as W7).

$$\text{Degree of wear} = (\text{Weight of catalyst before test (W6)} - \text{weight of catalyst after test (W7)})/\text{weight of catalyst before test (W6)} \times 100$$

(Bulk specific gravity/graduated cylinder method)

Method of measurement:

1. 50 ml of a catalyst is measured (W5) in a 100-ml graduated cylinder having an internal diameter of 28.7 mm.

$$\text{Bulk specific gravity} = W5/50$$

(Porosity/graduated cylinder method)

Method of measurement:

1. 50 ml of a catalyst is measured in a 100-ml graduated cylinder having an internal diameter of 28.7 mm.
2. 50 ml of pure water is added thereto, the pressure is reduced with a water aspirator, and the water is degassed until almost no bubbles come out. The degassing is carried out for a degassing time of 1 minute to 2 minutes.
3. The water level after degassing (V1) is measured.

$$\text{Porosity (\%)} = (1 - (V1 - 50)/50) \times 100$$

$$\text{Conversion rate (\%)} = \text{(Number of moles of reacted propylene)/(number of moles of supplied propylene)} \times 100$$

$$\text{Selectivity ratio (\%)} = \text{(Number of moles of produced acrolein or acrylic acid)/(number of moles of reacted propylene)} \times 100$$

$$\text{Yield (\%)} = \text{(Number of moles of produced acrolein or acrylic acid)/(number of moles of supplied propylene)} \times 100$$

(Preparation of catalyst powder-1)

While 3000 parts of distilled water was heated and stirred, 423.8 parts of ammonium molybdate and 2.02 parts of potassium nitrate were dissolved therein, and thus an aqueous solution (A) was obtained. Separately, an aqueous solution (B) was prepared by dissolving 302.7 parts of cobalt nitrate, 162.9 parts of nickel nitrate, and 145.4 parts of ferric nitrate in 1000 parts of distilled water, and an aqueous solution (C) was prepared by dissolving 164.9 parts of bismuth nitrate in 200 parts of distilled water that had been acidified by adding 25 parts of concentrated nitric acid. The aqueous solutions (B) and (C) were mixed, and the mixed liquid was added dropwise to the aqueous solution (A) under vigorous stirring. The suspension liquid thus produced was dried using a spray dryer and was preliminarily calcined at 440°C for 3 hours, and thus 570 parts of a preliminarily calcined powder was obtained. The instant product was designated as catalyst powder-1. The composition excluding oxygen of the catalyst powder-1 was Mo =12, Bi =1.7, Ni = 2.8, Fe =1.8, Co = 5.2, and K = 0.1, in terms of atomic ratio.

Support-1

The method for producing a support-1 and the properties of the support are as follows.
500 g of a ring-shaped support having an outer circumferential radius of curvature of 0.0 mm, an outer diameter of 5.8 mm, a length of 6.5 mm, and an aperture diameter of 2.8 mm, was introduced into a ball mill having a capacity of 2000 ml and was rotated for 2 hours. The support thus obtained had an outer circumferential radius of curvature of 1.3 mm, an outer diameter of 5.8 mm, a length of 6.3 mm, and an aperture diameter of 2.8 mm. Furthermore, the support had a void content of 35%, a water absorption ratio of 15%, a bulk specific gravity of 1.01, and a porosity of 67%.

Support-2

The properties of a support-2 were measured, and the values shown below were obtained.
A ring-shaped support having an outer circumferential radius of curvature of 1.5 mm, an outer diameter of 5.8 mm, a length of 6.4 mm, and an aperture diameter of 1.3 mm, had a void content of 33%, a water absorption ratio of 14%, a bulk specific gravity of 1.01, and a porosity of 67%.

Support-3

The method for producing a support-3 and the properties of the support are as follows.
400 g of a ring-shaped support having an outer circumferential radius of curvature of 0.0 mm, an outer diameter of 6.4 mm, a length of 5.3 mm, and an aperture diameter of 2.0 mm, was subjected to planing of the outer periphery with a tumbling granulator having a diameter of 30 cm, for one hour at a speed rotation of 260 rpm. The support thus obtained had an outer circumferential radius of curvature of 0.5 mm, an outer diameter of 6.3 mm, a length of 5.1 mm, and an aperture diameter of 2.0 mm. Furthermore, the support had a void content of 46%, a water absorption ratio of 24%, a bulk specific gravity of 0.83, and a porosity of 75%.

Support-4

The properties of a support-4 were measured, and the values shown below were obtained.

The support had an outer circumferential radius of curvature of 1.0 mm, an outer diameter of 5.8 mm, a length of 6.5 mm, and an aperture diameter of 1.8 mm. Furthermore, the support had a void content of 48%, a water absorption ratio of 25%, a bulk specific gravity of 0.81, and a porosity of 78%.

Support-5 (Comparative Example)

The properties of the ring support used as the raw material of the support-1 were measured, and the values shown below were obtained.

Outer circumferential radius of curvature 0.0 mm, outer diameter 5.8 mm, length 6.5 mm, aperture diameter 2.8 mm, void content 35%, water absorption ratio 16%, bulk specific gravity 0.98, and porosity 66%.

Example 1

Preparation of catalyst-1

300 ml of the ring-shaped support-1 was introduced into a tumbling granulator, and was wetted with an aqueous glycerin solution. Subsequently, a mixture of 300 g of the catalyst powder-1 and 15 g of crystalline cellulose was added thereto alternately with an aqueous glycerin solution, and thus catalyst powder-supported particles were prepared. Thereafter, these particles were dried at room temperature for 15 hours, and then were calcined at 530°C for 5 hours under an air stream, and thus a catalyst of the present invention was obtained. The catalyst thus obtained had an outer diameter of 7.4 mm, a length of 7.7 mm, and an aperture diameter of 1.8 mm. Furthermore, the catalyst had a degree of wear of 4.0%, a bulk specific gravity of 1.03, and a porosity of 68%.

Example 1-2

Preparation of catalyst-1-2

The granulation chamber was regulated to have a weight absolute humidity of 0.015 (chamber temperature 25°C), and 300 ml of the ring-shaped support-1 was introduced into the tumbling granulator and was wetted with an aqueous glycerin solution. Subsequently, a mixture of 300 g of the catalyst powder-1 and 15 g of crystalline cellulose was added thereto alternately with an aqueous glycerin solution, and thus catalyst powder-supported particles were prepared. Thereafter, these particles were dried at room temperature for 15 hours, and then were calcined at 530°C for 5 hours under an air stream, and thus a catalyst of the present invention was obtained. The catalyst thus obtained had an outer diameter of 7.5 mm, a length of 7.6 mm, and an aperture diameter of 0.5 mm. Furthermore, the catalyst had a degree of wear of 1.2%, a bulk specific gravity of 1.03, and a porosity of 66%.

Example 2

Preparation of catalyst-2

A catalyst of the present invention was obtained in the same manner as in Example 1, except that the ring-shaped support-2 was used as the support. The catalyst thus obtained had an outer diameter of 7.1 mm, a length of 7.7 mm, and an aperture diameter of 1.1 mm. Furthermore, the catalyst had a degree of wear of 3.0%, a bulk specific gravity of 1.06, and a porosity of 70%.

Example 2-2

Preparation of catalyst-2-2

A catalyst of the present invention was obtained in the same manner as in Example 1, except that the granulation chamber was regulated to have a weight absolute humidity of 0.02 (chamber temperature 30°C), and the ring-shaped support-2 was used as the support. The catalyst thus obtained had an outer diameter of 7.4 mm, a length of 7.6 mm, and an aperture diameter of 0.1 mm. Furthermore, the catalyst had a degree of wear of 0.6%, a bulk specific gravity of 1.06, and a porosity of 68%.

Example 3

Preparation of catalyst-3

A catalyst of the present invention was obtained in the same manner as in Example 1, except that the ring-shaped support-3 was used as the support. The catalyst thus obtained had an outer diameter of 7.9 mm, a length of 6.9 mm, and an aperture diameter of 0.5 mm. Furthermore, the catalyst had a degree of wear of 5.0%, a bulk specific gravity of 1.04, and a porosity of 72%.

Example 3-2

Preparation of catalyst-3-2

A catalyst of the present invention was obtained in the same manner as in Example 1, except that the granulation chamber was regulated to have a weight absolute humidity of 0.037 (chamber temperature 35°C), and the ring-shaped support-3 was used as the support. The catalyst thus obtained had an outer diameter of 7.9 mm, a length of 6.7 mm, and an aperture diameter of 0.1 mm. Furthermore, the catalyst had a degree of wear of 1.8%, a bulk specific gravity of 1.05, and a porosity of 71%.

Example 4

Preparation of catalyst-4

A catalyst of the present invention was obtained in the same manner as in Example 1, except that the ring-shaped support-4 was used as the support. The catalyst thus obtained had an outer diameter of 7.4 mm, a length of 7.6 mm, and an aperture diameter of 1.5 mm. Furthermore, the catalyst had a degree of wear of 4.0%, a bulk specific gravity of 0.92, and a porosity of 76%.

Example 4-2

Preparation of catalyst-4-2

A catalyst of the present invention was obtained in the same manner as in Example 1, except that the granulation chamber was regulated to have a weight absolute humidity of 0.015 (chamber temperature 27°C), and the ring-shaped support-4 was used as the support. The catalyst thus obtained had an outer diameter of 7.5 mm, a length of 7.5 mm, and an aperture diameter of 0.2 mm. Furthermore, the catalyst had a degree of wear of 0.8%, a bulk specific gravity of 0.92, and a porosity of 75%.

Example 4-3

Preparation of catalyst-4-3

A catalyst of the present invention was obtained in the same manner as in Example 1, except that the granulation chamber, drying process and calcination process were humidified to a weight absolute humidity of 0.015 (chamber temperature 27°C), and the ring-shaped support-4 was used as the support. The catalyst thus obtained had an outer diameter of 7.6 mm, a length of 7.5 mm, and an aperture diameter of 0.2 mm. Furthermore, the catalyst had a degree of wear of 0.6%, a bulk specific gravity of 0.92, and a porosity of 75%.

Comparative Example 1

(Preparation of catalyst-5)

A catalyst was obtained in the same manner as in Example 1, except that the ring-shaped support-5 was used as the support. The catalyst thus obtained had an outer diameter of 7.8 mm, a length of 7.8 mm, and an aperture diameter of 1.5 mm. Furthermore, the catalyst had a degree of wear of 26.0%, a bulk specific gravity of 0.85, and a porosity of 74%.

Reaction Example 1

A stainless steel (SUS304) reaction tube having a total length of 50 cm and an internal diameter of 28.4 mm was perpendicularly installed, and a thermocouple having an external diameter of 3.2 mm was installed at the center of the reaction tube. The catalyst-1 was packed in the reaction tube to a height of 15 cm, and then ceramic balls that were inert to the reaction were packed above the catalyst and to the top of the reaction tube.

The reaction bath temperature was maintained at 320°C, and a mixed gas formed from 8.3% by volume ofpropylene (propylene flow rate 4.82 L/hr), 14.0% by volume of oxygen, 24.8% by volume of water vapor, and 52.9% by volume of nitrogen, was passed over the catalyst to react.

At this time, the maximum temperature of the catalyst layer was 397°C, the propylene conversion rate was 97.2%, the total yield of acrolein and acrylic acid was 91.6%, and the total selectivity ratio for acrolein and acrylic acid was 94.2%.

Reaction Example 1-2

The reaction was carried out in the same manner as in Reaction Example 1, except that the catalyst used in the Reaction Example 1 was changed to the catalyst-1-2.

At this time, the maximum temperature of the catalyst layer was 398°C, the propylene conversion rate was 97.4%, the total yield of acrolein and acrylic acid was 91.7%, and the total selectivity ratio for acrolein and acrylic acid was 94.1%.

Reaction Example 2

The reaction was carried out in the same manner as in Reaction Example 1, except that the catalyst used in the Reaction Example 1 was changed to the catalyst-2.

At this time, the maximum temperature of the catalyst layer was 395°C, the propylene conversion rate was 97.1%, the total yield of acrolein and acrylic acid was 91.4%, and the total selectivity ratio for acrolein and acrylic acid was 94.1%.

Reaction Example 2-2

The reaction was carried out in the same manner as in Reaction Example 1, except that the catalyst used in the Reaction Example 1 was changed to the catalyst-2-2.

At this time, the maximum temperature of the catalyst layer was 397°C, the propylene conversion rate was 97.2%, the total yield of acrolein and acrylic acid was 91.4%, and the total selectivity ratio for acrolein and acrylic acid was 94.0%.

Reaction Example 3

The reaction was carried out in the same manner as in Reaction Example 1; except that the catalyst used in the Reaction Example 1 was changed to the catalyst-3.

At this time, the maximum temperature of the catalyst layer was 392°C, the propylene conversion rate was 95.9%, the total yield of acrolein and acrylic acid was 89.0%, and the total selectivity ratio for acrolein and acrylic acid was 92.8%.

Reaction Example 3-2

The reaction was carried out in the same manner as in Reaction Example 1, except that the catalyst used in the Reaction Example 1 was changed to the catalyst-3-2.

At this time, the maximum temperature of the catalyst layer was 392°C, the propylene conversion rate was 95.9%, the total yield of acrolein and acrylic acid was 89.0%, and the total selectivity ratio for acrolein and acrylic acid was 92.8%.

Reaction Example 4

The reaction was carried out in the same manner as in Reaction Example 1, except that the catalyst used in the Reaction Example 1 was changed to the catalyst-4.

At this time, the maximum temperature of the catalyst layer was 390°C, the propylene conversion rate was 96.5%, the total yield of acrolein and acrylic acid was 91.1%, and the total selectivity ratio for acrolein and acrylic acid was 94.4%.

Reaction Example 4-2

The reaction was carried out in the same manner as in Reaction Example 1, except that the catalyst used in the Reaction Example 1 was changed to the catalyst-4-2.
At this time, the maximum temperature of the catalyst layer was 391 °C, the propylene conversion rate was 96.5%, the total yield of acrolein and acrylic acid was 91.1 %, and the total selectivity ratio for acrolein and acrylic acid was 94.4%.

Reaction Example 4-3

The reaction was carried out in the same manner as in Reaction Example 1, except that the catalyst used in the Reaction Example 1 was changed to the catalyst-4-3.
At this time, the maximum temperature of the catalyst layer was 390°C, the propylene conversion rate was 96.4%, the total yield of acrolein and acrylic acid was 91.0%, and the total selectivity ratio for acrolein and acrylic acid was 94.4%.

Reaction Example 5: Comparative Example

The reaction was carried out in the same manner as in Reaction Example 1, except that the catalyst used in the Reaction Example 1 was changed to the catalyst-5.
At this time, the maximum temperature of the catalyst layer was 390°C, the propylene conversion rate was 93.8%, the total yield of acrolein and acrylic acid was 88.3%, and the total selectivity ratio for acrolein and acrylic acid was 94.1%.
As such, the catalyst of the present invention is a catalyst which exhibits high activity and high yield, and has an excellent degree of wear.

**Claims**

1. A catalyst for producing an unsaturated aldehyde and an unsaturated carboxylic acid by correspondingly subjecting propylene, isobutylene, tertiary-butyl alcohol or methyl tertiary-butyl ether to gas phase catalytic oxidation, the catalyst being a supported catalyst having an inert support coated with a catalyst powder, wherein the inert support is ring-shaped and has an outer periphery that is curved in the lengthwise direction of the support.

2. A catalyst for producing an unsaturated carboxylic acid by subjecting a corresponding unsaturated aldehyde to gas phase catalytic oxidation, the catalyst being a supported catalyst having an inert support coated with a catalyst powder, wherein the inert support is ring-shaped and has an outer periphery that is curved in the lengthwise direction of the support.

3. A method of producing a catalyst for producing an unsaturated aldehyde and an unsaturated carboxylic acid by correspondingly subjecting propylene, isobutylene, tertiary-butyl alcohol or methyl tertiary-butyl ether to gas phase catalytic oxidation, the method comprising a step of coating an inert support with a catalyst powder to produce a supported catalyst, wherein the inert support is ring-shaped and has an outer periphery that is curved in the lengthwise direction of the support, and wherein the catalyst is produced in an atmosphere of the step of coating that has been regulated to have a weight absolute humidity of 0.01 or higher.

4. A method of producing a catalyst for producing an unsaturated carboxylic acid by subjecting a corresponding unsaturated aldehyde to gas phase catalytic oxidation, the method comprising a step of coating an inert support with a catalyst powder to produce a supported catalyst, wherein the inert support is ring-shaped and has an outer periphery that is curved in the lengthwise direction of the support, and wherein the catalyst is produced in an atmosphere of the step of coating that has been regulated to have a weight absolute humidity of 0.01 or higher.

5. A method of producing an unsaturated aldehyde and an unsaturated carboxylic acid,
   wherein propylene, isobutylene, tertiary-butyl alcohol or methyl tertiary-butyl ether is correspondingly subjected to gas phase catalytic oxidation, by using the catalyst according to claim 1 or the catalyst obtainable by the method according to claim 3.

6. A method of producing an unsaturated carboxylic acid, wherein an unsaturated aldehyde is correspondingly subjected to gas phase catalytic oxidation by using the catalyst according to claim 2 or the catalyst obtainable by the method according to claim 4.

## INTERNATIONAL SEARCH REPORT

| | International application No. |
|---|---|
| | PCT/JP2009/059503 |

A.  CLASSIFICATION OF SUBJECT MATTER
*B01J35/02*(2006.01)i, *B01J23/88*(2006.01)i, *B01J37/00*(2006.01)i, *C07C27/12*
(2006.01)i, *C07C45/35*(2006.01)i, *C07C47/22*(2006.01)i, *C07C57/05*(2006.01)i,
*C07B61/00*(2006.01)n, *C07C51/235*(2006.01)n, *C07C51/25*(2006.01)n
According to International Patent Classification (IPC) or to both national classification and IPC

B.  FIELDS SEARCHED

Minimum documentation searched (classification system followed by classification symbols)
B01J35/02, B01J23/88, B01J37/00, C07C27/12, C07C45/35, C07C47/22,
C07C57/05, C07B61/00, C07C51/235, C07C51/25

Documentation searched other than minimum documentation to the extent that such documents are included in the fields searched
Jitsuyo Shinan Koho        1922-1996   Jitsuyo Shinan Toroku Koho   1996-2009
Kokai Jitsuyo Shinan Koho   1971-2009   Toroku Jitsuyo Shinan Koho   1994-2009

Electronic data base consulted during the international search (name of data base and, where practicable, search terms used)
JSTPlus(JDreamII), JST7580(JDreamII)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 61-141933 A  (BASF AG.),<br>28 June, 1986 (28.06.86),<br>Claim (1); page 3, upper left column, line 4 to<br>upper right column, line 7; Figs. 1 to 2<br>& US 4656157 A          & EP 184790 A2 | 1-2,5-6<br>3-4 |
| Y<br>A | JP 2001-079408 A  (Nippon Kayaku Co., Ltd.),<br>27 March, 2001 (27.03.01),<br>Claims 1 to 3; example 3<br>& US 2003/0109381 A1     & EP 1138385 A1<br>& WO 2001/021307 A1 | 3-4<br>1-2,5-6 |
| A | JP 2001-293376 A  (BASF AG.),<br>23 October, 2001 (23.10.01),<br>Claim 1; Par. Nos. [0005], [0010]; Figs. 1 to 3<br>& US 2001/0029235 A1     & EP 1127618 A1 | 1-6 |

☒  Further documents are listed in the continuation of Box C.          ☐  See patent family annex.

| | |
|---|---|
| *      Special categories of cited documents:<br>"A"    document defining the general state of the art which is not considered   to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| 08 June, 2009 (08.06.09) | 16 June, 2009 (16.06.09) |

| Name and mailing address of the ISA/<br>Japanese Patent Office | Authorized officer |
|---|---|
| Facsimile No. | Telephone No. |

Form PCT/ISA/210 (second sheet) (April 2007)

**INTERNATIONAL SEARCH REPORT**

| International application No. |
| --- |
| PCT/JP2009/059503 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 1999/019065 A1 (Japan Energy Corp.), 22 April, 1999 (22.04.99), Claim 1; page 4, lines 7 to 8; page 5, lines 22 to 25; Figs. 1A to 3B & US 6325919 B1      & EP 1052018 A1 | 1-6 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6170232 A
- JP 2001293376 A
- JP 2002273229 A
- JP 61141933 A
- JP 2008207127 A
- JP 10028877 A
- JP 50030815 A
- JP 2001079408 A
- JP 55079341 A
- JP 55122734 A